# EUROPÄISCHE PATENTSCHRIFT

(11) **EP 0 845 998 B1**
(45) Veröffentlichungstag und Bekanntmachung des Hinweises auf die Patenterteilung: **14.11.2001**
(21) Anmeldenummer: 96929318.2
(22) Anmeldetag: 23.08.1996
(51) Int. Cl.: A61K 39/395, C07K 16/46, C07K 16/28, G01N 33/577

(54) **ARZNEIMITTEL ZUR IMMUNTHERAPIE, ENTHALTEND ANTIKÖRPER, DIE SPEZIFISCH DAS MHCII-ANTIGEN EINES ZU BEHANDELNDEN PATIENTEN ERKENNEN**
IMMUNOTHERAPY MEDICAMENTS CONTAINING ANTIBODIES WHICH SPECIFICALLY DETECT THE MHCII ANTIGEN OF A PATIENT TO BE TREATED
MEDICAMENTS S'UTILISANT EN IMMUNOTHERAPIE ET CONTENANT DES ANTICORPS IDENTIFIANT SPECIFIQUEMENT L'ANTIGENE DU MHCII D'UN PATIENT A TRAITER

(30) Priorität: 25.08.1995 DE 19531346
(43) Veröffentlichungstag der Anmeldung: 10.06.1998
(73) Patentinhaber: GSF - Forschungszentrum für Umwelt und Gesundheit GmbH, 85764 Oberschleissheim (DE)
(72) Erfinder: LINDHOFER, Horst, D-82194 Gröbenzell (DE); THIERFELDER, Stefan, D-82223 Eichenau (DE)
(74) Vertreter: VOSSIUS & PARTNER
(86) Internationale Anmeldenummer: EP9603733
(87) Internationale Veröffentlichungsnummer: WO9707819

(56) Entgegenhaltungen:
- EP-A- 0 068 790
- CA-A- 1 329 545
- US-A- 4 676 980
- STEM CELLS, Bd. 13, Nr. 2, März 1995, DAYTON, OH, VSA, Seiten 123-134, XP000616204 Y. HUANG ET AL.: "Immunotherapy of multiple myeloma."
- THE FASEB JOURNAL, Bd. 6, Nr. 5, 28.Februar 1992, BETHESDA, MD, VSA, Seite A2060 XP002024143 B. SEON ET AL.: "Four groups of new monoclonal antibodies which are directed to individually different cell surface antigens on human leukemia-lymphoma cells and whose conjugates with ricin A chain are effective for specific tumor cell killing."
- THE JOURNAL OF IMMUNOLOGY, Bd. 150, Nr. 6, 15.März 1993, BALTIMORE, MD, USA, Seiten 2211-2221, XP002024144 S. WU ET AL.: "Use of bispecific heteroconjugated antibodies (anti-T cell antigen receptor x anti-MHC class II) to study activation of T cells with a full length or truncated antigen receptor zeta-chain."
- THE JOURNAL OF IMMUNOLOGY, Bd. 152, Nr. 8, 15.April 1994, BALTIMORE, MD, USA, Seiten 3833-3841, XP002024145 J. YAGI ET AL.: "Superantigen-like prperties of an antibody bispecific for MHC class II molecules and the Vbeta domain of the T cell antigen receptor."
- JOURNAL OF INVESTIGATIVE MEDICINE, Bd. 43, Nr. suppl. 3, September 1995, Seite 482A XP000616209 B. LINK ET AL.: "Humanized BsF(ab')2 anti-CD3-based bispecific monoclonal antibody designed for therapy of B cell malignancies."

## Beschreibung

Die vorliegende Erfindung betrifft die Verwendung eines monospezifischen Antikörpers, des spezifisch das MHCII-Antigen eines zu behandelnden Patienten erkennt, oder eines Antikörpers mit zwei oder mehr Spezifitäten, wobei mindestens eine spezifität das MHCII-Antigen eines zu behandelnden Patienten erkennt, zur Herstellung eines Arzneimittels zur zur eliminierung residuelles Tumorzellen nach einer MHCII-inkompatiblen Knochenmarktransplantation (KMT).

Die Knochenmarktransplantation bietet sich als eine aussichtsreiche Therapie an für Leukämiepatienten, bei denen es trotz Chemotherapie zum Leukämierückfall kommt. Mit Hilfe von Knochenmarktransplantationen ist es möglich, Langzeitremissionen und Heilung herbeizuführen, so daß Patienten bereits 20 Jahre und länger leben und als geheilt angesehen werden können. Nach wie vor sterben jedoch nach Knochenmarktransplantationen viele behandelte Patienten am Leukämierezidiv, der Metastasierung der ursprünglichen Leukämie. Dies beruht offensichtlich darauf, daß Ganzkörperbestrahlung und hochdosierte Chemotherapie, die dem Ersatz der verlorengegangenen Blutbildung des Patienten durch Transplantation von gesundem Knochenmark vorausgehen, häufig nicht ausreichen, um auch die letzten malignen Leukämiezellen dauerhaft zu eliminieren.
Innerhalb der letzten 14 Jahre wurden keine wesentlichen Fortschritte bei der Reduktion des Anteils der Patienten mit Leukämierückfällen nach Knochenmarktransplantation erreicht. Die bisherigen Therapiemöglichkeiten scheinen in dieser Beziehung ausgeschöpft. Neue Ansätze für Therapiemöglichkeiten zur Eliminierung residualer, trotz Chemotherapie und Bestrahlung überdauernder Leukämiezellen erwägen insbesondere eine tumorimmunologische Zelltherapie, bei der Antikörper verwendet werden sollten, die Zellmarker erkennen, die spezifisch auf Leukämiezellen vorhanden sind.

Die therapeutische Verwendung von Antikörpern ist generell im Stand der Technik bekannt. Beispielsweise wird in der EP-A2 0 068 790 der Einsatz von monoklonalen anti-MHC Antikörpern zur Behandlung von Autoimmunerkrankungen beschrieben. Huang und Vitetta (Stem Cells 13 (1995), 123-134) berichten von der inhibitorischen Wirkung von anti-MHCII Antikörpern auf das Wachstum von Myeloma-Zellinien in vitro. Außerdem wird in dem US-Patent 4,676,980 die Verwendung von Heteroantikörpern zur selektiven Zerstörung einer Zielzelle beschrieben, die aus einem Antikörper gegen eine T-Zell-Determinante und einem Antikörper gegen eine Oberflächenstruktur der Zielzelle bestehen. Ein wesentliches bisher nicht gelöstes Problem besteht bei der Eliminierung residualer Leukämiezellen mittels tumorimmunologischer Zelltherapie jedoch darin,
daß keine tumorspezifischen Markerantigene identifiziert werden konnten, aufgrund derer sich Leukämiezellen von nicht-malignen Blutzellen unterscheiden lassen (Kranz et al., Blood 73 (1989), 1942). Lediglich B-Zelltumore bilden individualspezifische, idiotypische Immunglobuline, die (auf den Einzelpatienten bezogen) als tumorspezifische Antigene angesehen werden können. Wie Untersuchungen mit chemisch konjugierten bispezifischen Antikörpern zeigten, neigen diese zellständigen Immunglobuline jedoch zu Modulation und Mutation, wodurch sie sich der Erkennung durch spezifisch gegen sie gerichtete Antikörper entziehen (Stevenson et al., Blood 77 (1991), 1071). Die bisherigen Kenntnisse reichen daher nicht aus, um verbliebene Leukämiezellclone durch eine immunologische Zelltherapie auszuschalten.

Der vorliegenden Erfindung liegt somit die Aufgabe zugrunde, Antikörper zur Verfügung zu stellen, die es erlauben, bestimmte Zielzellen, insbesondere Tumorzellen, von anderen Zellen aufgrund der Erkennung spezifischer Markerantigene zu unterscheiden, und die sich somit für eine immunologische Zelltherapie eignen.

Diese Aufgabe wird durch die Bereitstellung der in den Patentansprüchen bezeichneten Ausführungsformen gelöst.

Die vorliegende Erfindung betrifft somit die Verwendung eines monospezifischen Antikörpers, der spezifisch das MHCII-Antigen eines zu behandelnden Patienten erkennt, oder eines Antikörpers mit zwei oder mehr Spezifitäten, wobei mindestens eine Spezifität das MHCII-Antigen eines zu behandelnden Patienten erkennt, zur Herstellung eines Arzneimittels zur Eliminierung residueller Tumorzellen nach einer MHCII-inkompatiblen Knochenmarkstransplantation.

Derartige Arzneimittel eignen sich auch zur in vivo- und in vitro-Therapie verschiedener Tumorarten sowie zur Unterdrückung von Host-versus-Graft-Reaktionen, Autoimmunreaktionen und zur generellen Auslösung einer Immunsuppression. So können sie beispielsweise zur immunologischen Zelltherapie bei Tumorrezidiven nach einer MHCII-inkompatiblen Knochenmarktransplantation, d.h. einer Transplantation, bei der sich das MHCII-Antigen des Spenders von dem des Empfängers unterscheidet, eingesetzt werden. Wie oben beschrieben wird die Knochenmarktransplantation häufig als letztes Mittel gegen chemotheraphieresistente Leukämieformen eingesetzt. Bei diesen exprimieren vor allem B-Zell-Lymphome, aber auch andere Leukämieformen, die sich von Zellen ableiten, die aus der Hämatopoese hervorgehen, häufig das Histokompatibilitätsantigen MHCII. Dieses Oberflächenantigen ist hochpolymorph, wobei die unterschiedlichen Formen von spezifischen Antikörpern erkannt werden können. Da nach einer MHCII-inkompatiblen Knochenmarktransplantation alle in dem Patienten heranreifenden immunkompetenten (vom Spender stammenden) Zellen einen MHCII-Typ besitzen, der von dem des Tumors abweicht, kann der Tumor sehr selektiv durch Antikörper, die den MHCII-Typ des Empfängers erkennen, identifiziert und eliminiert werden. Zwar existieren neben den Zellen des Immunsystems andere Zelltypen, die MHCII-Antigen exprimieren, wie z.B. Leberzellen, jedoch ist die Expression und die Antigendichte auf der Zelloberfläche bei diesen Zelltypen im Vergleich zu malignen Zellen des Immunsystems derart gering, daß eine spezifische Erkennung der Tumorzellen, die MHCII-Antigen exprimieren, gewährleistet ist und andere Zellen oder Gewebe nicht nennenswert durch MHCII-spezifische Antikörper geschädigt werden.
Neben hämatopoetischen Zellen des Immunsystems exprimieren auch Zellen des Epithels (wie z.B. Langerhanssche-insulinbildende Inselzellen im Pankreas) und Zellen des Endothels in unterschiedlicher Ausprägung, insbesondere in entartetem Zustand, oder auch nach Stimulierung das MHCII-Antigen. Die vorgeschlagenen Arzneimittel bieten sich daher auch zur Immuntherapie von Tumoren an, die sich von derartigen Zellen ableiten.

Neben der Anwendung zur Tumorimmuntherapie eignen sich die erfindungsgemäßen Arzneimittel beispielsweise auch zur Immuntherapie, um eine Host-versus-Graft-Reaktion abzuschwächen oder zu unterdrücken. Nach allogener Knochenmarktransplantation können restliche, die Konditionierung (z.B. Bestrahlung, Chemotherapie) des Patienten überlebende, MHCII-positive Zellen des Empfängers zur Abstoßung des Transplantats in Form einer Host-versus-Graft-Reaktion führen. Durch anti-MHCII (vom Empfängertyp)-Antikörper können derartige immunkompetente Zellen des Empfängers, die das "Engraftment" (d.h. Anwachsen, Angehen) des neuen Knochenmarks oder anderer Spenderorgane verhindern, gezielt attackiert werden.
Durch Verwendung von Antikörpern, die neben dem MHCII-Antigen des Empfängers ein Antigen erkennen, das auf einer immunkompetenten MHCII-positiven Zelle exprimiert wird, können für die Abstoßung des Transplantats verantwortliche Zellen noch selektiver ausgeschaltet werden.
Arzneimittel, die Antikörper enthalten, die spezifisch aktivierte T-Zellen erkennen, eignen sich beispielsweise für die Behandlung von Autoimmunreaktionen oder generell für eine gezielte Immunsuppression.

Bei den Antikörpern, die zur Herstellung eines erfindungsgemäßen Arzneimittels verwendet werden, kann es sich um Antikörper mit lediglich einer oder mehreren Spezifitäten handeln. Der Ausdruck Spezifität bedeutet dabei die Fähigkeit eines Antikörpers, ein bestimmtes Antigen zu erkennen. Das Antigen kann dabei von einer oder mehreren Antigenbindungsstellen des Antikörpers erkannt werden, die wiederum gleiche oder unterschiedliche Epitope des Antigens erkennen können.

Zur Herstellung der Arzneimittel werden Antikörper mit lediglich einer Spezifität, die das MHCII-Antigen eines zu behandelnden Patienten erkennen, d.h. sogenannte monospezifische Antikörper verwendet. Durch die Verwendung von derartigen Antikörpern kann eine Eliminierung von MHCII-präsentierenden Zellen beispielsweise durch Komplement-Reaktionen oder antikörpervermittelter Zytotoxizität mittels Fc-Rezeptor-tragender Zellen bewirkt werden. Voraussetzung hierfür ist, daß die verwendeten Antikörper eine Fc-Region besitzen.

Alternativ werden Antikörper mit zwei oder mehr Spezifitäten verwendet, wovon eine das MHCII-Antigen eines Patienten erkennt. Eine Spezifität erkennt dabei das MHCII-Antigen des Patienten, wohingegen eine weitere Spezifität z.B. ein Antigen erkennen kann, das ebenfalls auf der zu eliminierenden Zelle vorhanden ist, ein Antigen, das auf einer anderen Zelle, beispielsweise einer Effektorzelle lokalisiert ist, oder ein beliebiges anderes Antigen. Hierbei sind solche zu nennen, die die Effektorfunktion des Antikörpers verstärken, d.h. dessen Fähigkeit, die Antigentragende Zelle zu eliminieren. Dazu zählen z.B. toxische Substanzen, wie weiter unten erläutert wird.
Die Verwendung von Antikörpern mit mehr als einer Spezifität in den erfindungsgemäßen Arzneimitteln bietet sich beispielsweise an, um die Selektivität zu erhöhen, mit der die Antikörper eine zu eliminierende Zielzelle, beispielsweise eine Tumorzelle, im Vergleich zu anderen Zellen erkennen. Wie bereits oben erläutert, wird das MHCII-Antigen zum Teil auch von nicht-malignen Zellen, z.B. Leberzellen, auf der Zelloberfläche präsentiert, wenn auch in wesentlich geringerer Dichte. Um die Spezifität der verwendeten Antikörper für MHCII-exprimierende Tumorzellen zu erhöhen, können beispielsweise Antikörper eingesetzt werden, die zwei verschiedene auf der zu eliminierenden Zelle lokalisierte Antigene erkennen. Es handelt sich dabei um sogenannte bispezifische Antikörper. Bevorzugt werden dabei Antikörper verwendet, die neben dem MHCII-Antigen des zu behandelnden Patienten ein tumorassoziiertes Antigen erkennen.

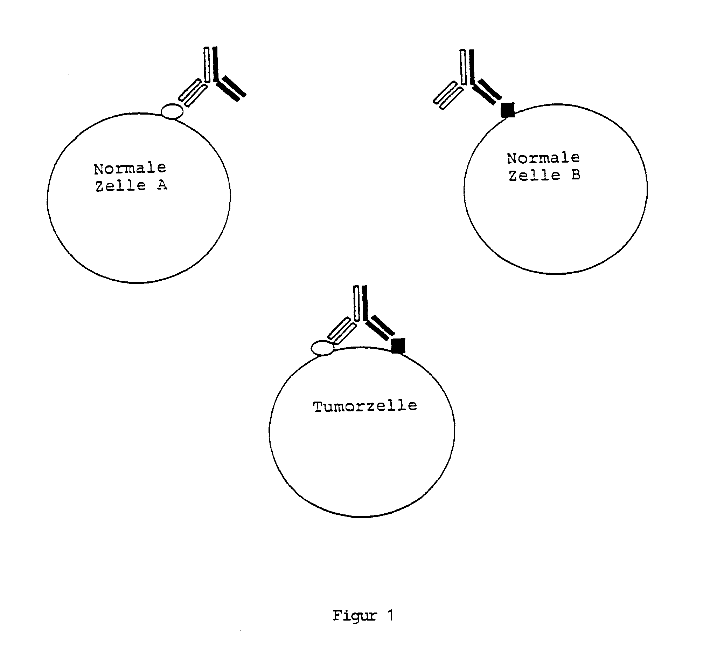

Der Begriff tumorassoziiertes Antigen bedeutet dabei, daß ein derartiges Antigen im Vergleich zu nicht-malignen Zellen bevorzugt von Zellen des zu behandelnden Tumors exprimiert wird, und/oder auf nicht-malignen Zellen in geringerer Dichte vorkommt.
Bei dem zweiten Antigen kann es sich ferner um ein Antigen handeln, das spezifisch von dem Gewebe, von dem der Tumor abstammt, exprimiert wird.
Gegenüber monospezifischen Antikörpern, die nur das MHCII-Antigen oder nur ein tumorassoziiertes oder gewebespezifisches Antigen erkennen, weisen derartige bispezifische Antikörper den Vorteil auf, daß sie an Zellen, die beide Antigene präsentieren, mit wesentlich höherer Affinität und Spezifität binden, als an Zellen, die lediglich eines der beiden Antigene präsentieren (Parham; Human Immunol. 12 (1985), 213; Wong und Colvin, J. Immunol. 139 (1987), 1369; siehe auch Figur 1).

In einer bevorzugten Ausführungsform handelt es sich bei dem Antigen, das neben MHCII von einem MHCII-spezifischen Antikörper mit zwei oder mehreren Spezifitäten erkannt wird, um ein Antigen, das auf Leukämiezellen, Zellen der Hämatopoese oder entarteten Zellen des Endothels oder Epithels exprimiert wird. Vorzugsweise handelt es sich dabei um das Antigen CD1, CD2, CD3, CD4, CD5, CD6, CD7, CD8, CD10, CD11, CD11b, CD13, CD14, CD19, CD20, CD21, CD22, CD23, CD24, CD30, CD33, CD37, CD40, CD41, CD44v3, CD44v6, CD45R, CD56, CD71, B220, einen Ig-Idiotyp, einen IL-2-Rezeptor oder einen IL-6-Rezeptor. Für die spezifische Erkennung von B-Zell-Lymphomen sind beispielsweise Antikörper geeignet, die neben dem MHCII-Antigen die Antigene CD19, CD20, CD21, CD22, CD23, CD24, CD44v3, CD44v6, CD10, CD5, B220 oder einen Ig-Idiotyp erkennen. Antigene wie CD1, CD2, CD3, CD4, CD5, CD6, CD7 und CD8 können spezifisch T-Zell-Lymphomen zugeordnet werden. Die Antigene CD11, CD13, CD14 und CD33 ermöglichen die spezifische Erkennung von myeloischen Leukämien, das Antigen CD41 hingegen von thrombozytären Leukämien. Dagegen bieten sich für die spezifische Erkennung von Zellen eines Hodgkin-Lymphoms, eines anaplastischen "large cell" Lymphoms oder einer adulten T-Zell-Leukämie Arzneimittel an, die Antikörper enthalten, die neben dem MHCII-Antigen des zu behandelnden Patienten einen IL-2-Rezeptor erkennen. Für die spezifische Erkennung von Myelomen bieten sich ferner Arzneimittel an, bei denen die Antikörper neben dem MHCII-Antigen des zu behandelnden Patienten einen IL-6-Rezeptor erkennen.

Die Eliminierung von Zellen, insbesondere von Tumorzellen, die durch die beschriebenen Antikörper erkannt werden, kann in vivo durch Komplement-Reaktionen oder durch antikörpervermittelte Zytotoxizität mittels Fc-Rezeptor-tragender Zellen erfolgen.

In einer weiteren bevorzugten Ausführungsform weist des Antikörper mit zwei oder mehr Spezifitäten mindestens eine weitere Spezifität auf, die ein Antigen, das spezifisch auf einer Effektorzelle lokalisiert ist, erkennt. Daneben kann ein derartiger Antikörper weitere Spezifitäten besitzen, wie unten noch weiter ausgeführt wird.
Unter dem Begriff Effektorzelle werden dabei Zellen verstanden, die aus der Hämatopoese hervorgehen und zytolytische, Apoptose-vermittelnde oder Phagozytose-Eigenschaften besitzen. Insbesondere umfaßt dieser Begriff T-Zellen, Granulocyten, Monocyten, Makrophagen, NK ("natural killer")-Zellen, Mastzellen und Langerhans-Zellen.
Bei dem auf der Effektorzelle lokalisierten Antigen, das von dem Antikörper erkannt wird, handelt es sich vorzugsweise um das Antigen CD1, CD2, CD3, CD4, CD5, CD8, CD11b, CD13, CD16, CD28, CD32, CD33, CD40L (auch bekannt als CD40 Ligand), CD44, CD45R, CD55, CD64 oder einen Interleukin-2-Rezeptor.

Mit Hilfe derartiger Antikörper ist es möglich, Effektorzellen zu aktivieren und/oder an Zielzellen heranzuführen, was somit zu deren Zerstörung führt.
Antikörper, die beispielsweise neben dem MHCII-Antigen eines zu behandelnden Patienten das Antigen CD3 erkennen, sind in der Lage, T-Zellen zu aktivieren und an die Zielzellen (MHCII-exprimierende Zellen) heranzuführen. Dies führt zur Zerstörung der Zielzellen über T-Zell-vermittelte Mechanismen. Antikörper, die das Antigen CD28 erkennen, das ebenfalls auf T-Zellen exprimiert wird und der Kostimulation während der Stimulation mittels CD3 dient, ermöglichen ebenfalls die Aktivierung von Effektorzellen. Ähnlich sind Antikörper, die die Antigene CD16, CD32 oder CD64 erkennen, in der Lage, Fc-Rezeptor-positive Zellen, wie z.B. NK-Zellen, Granulocyten oder Makrophagen, zu aktivieren und an die MHCII-tragenden Zielzellen, insbesondere Tumorzellen, heranzuführen. Dies führt im folgenden ebenfalls zur Zerstörung der Zielzellen.
Arzneimittel, die Antikörper enthalten, die neben dem Patienten-MHCII-Antigen das Antigen CD3, CD16, CD28, CD32 oder CD64 erkennen, eignen sich neben der oben beschriebenen Eliminierung von Zielzellen in vivo ebenso zur in-vitro-Anwendung zur Eliminierung von Tumorzellen, insbesondere bei allogener Knochenmarktransplantation.

Antikörper, die die Antigenkombinationen MHCII X CD1, MHCII X CD2, MHCII X CD4, MHCII X CD5, MHCII X CD6, MHCII X CD7, MHCII X CD8, MHCII X CD28, MHCII X CD40L, MHCII X CD45R oder MHCII X Interleukin-2-Rezeptor, MHCII X Interleukin-4-Rezeptor, MHCII x Interleukin-7-Rezeptor, hierbei insbesondere die Antigenkombinationen MHCII x γ-Kette des Interleukin-2-Rezeptors, des Interleukin-4-Rezeptors oder des Interleukin-7-Rezeptors, erkennen, sind ferner dafür geeignet, aktivierte T-Zellen zu erkennen und zu eliminieren, was im Zusammenhang mit der Unterdrückung einer Host-versus-Graft-Reaktion, einer Autoimmunreaktion oder der Auslösung einer generellen Immunsuppression von Bedeutung sein kann.

Für denselben Zweck eignen sich Antikörper, die die Antigenkombinationen MHCII X CD11b, MHCII X CD56 erkennen, die auf "Natural Killer"-Zellen exprimiert werden, sowie Antikörper, die die Antigenkombinationen MHCII X CD13, MHCII X CD14 oder MHCII X CD33 erkennen, die auf Monocyten und Makrophagen exprimiert werden.

In einer weiteren bevorzugten Ausführungsform weisen die in den beschriebenen Arzneimitteln enthaltenen Antikörper eine Spezifität, d.h. eine oder mehrere Bindungsstellen, auf, die in der Lage ist, spezifisch eine toxische Substanz zu binden, z.B. Saporin oder Ricin. Dies ermöglicht es, die Zielzellen selektiv und direkt durch das Toxin zu eliminieren.

Darüber hinaus sieht eine weitere bevorzugte Ausführungsform vor, daß die Antikörper mit Substanzen gekoppelt sind, die die Effektorfunktion der Antikörper, die zur Eliminierung der Zielzelle führen, verbessern.
Die zellzerstörende Wirkung von Antikörpern beruht normalerweise auf den Effektorfunktionen der Fc-Region der Antikörper. So können z.B. Fc-Rezeptor-tragende Zellen an eine mit Antikörpern besetzte Zelle binden und diese zerstören. Diese auch als ADCC ("antibody dependent cell mediated cytotoxicity") bezeichnete Reaktion reicht jedoch häufig nicht aus, um effektiv alle Zielzellen zu zerstören. Bei den Substanzen, die an die Antikörper gekoppelt werden können, handelt es sich vorzugsweise um Enzyme, toxische Substanzen, Biotin, Radionuclide oder Superantigene.

Im Zusammenhang mit der Kopplung des Antikörpers mit einem Enzym ist beispielsweise die von Rodrigues et al. (Cancer Res. 55 (1995), 63) beschriebene sogenannte "antibody dependent enzyme mediated prodrug therapy" (ADEPT) zu nennen. Bei diesem Verfahren wird die Vorstufe einer toxischen Substanz unmittelbar am vorgesehenen Wirkort, beispielsweise an der Tumorzelle, durch ein Enzym, das an den Antikörper gekoppelt ist, aktiviert. Dieses Prinzip ist umso effizienter und freier von Nebenwirkungen, je spezifischer der verwendete Antikörper die Zielzelle erkennt. Ein bevorzugt in dieser Methode verwendetes Enzym ist beispielsweise die β-Lactamase.

Beispiele für toxische Substanzen, die direkt an die verwendeten Antikörper gekoppelt werden können, sind Ricin, Saporin oder Pertussis-Toxin.

Durch die Kopplung des in dem Arzneimittel enthaltenen Antikörpers mit Biotin ist es ferner möglich, an Avidin konjugierte Substanzen an Zielzellen anzureichern.
Vorteilhaft ist dies beispielsweise bei der Applikation von Radionuclid-Avidin-Konjugaten. In diesem Fall werden die Konjugate erst verabreicht, wenn der mit Biotin gekoppelte Antikörper bereits an die Zielzelle gebunden hat. Die Konjugate werden aufgrund der hohen Affinität zwischen Biotin und Avidin sehr spezifisch an den Zielzellen angereichert. Ungebundene Radionuclid-Avidin-Konjugate werden dagegen aufgrund ihrer geringen Größe schnell aus dem Organismus entfernt. Der Vorteil der Verwendung derartiger Antikörper ist eine relativ geringe unspezifische radioaktive Belastung des Patienten bei ihrer Anwendung. Dieser Vorteil kann noch verstärkt werden, wenn Antikörper mit mehreren Spezifitäten, die eine hohe Zielzellselektivität aufweisen, verwendet werden.

In einer weiteren bevorzugten Ausführungsform sind die Antikörper gekoppelt mit einem Superantigen. Superantigene sind extrem potente Aktivatoren von T-Zellen. Ein Beispiel ist das SEA ("staphylococal enterotoxin A"). T-Zellen werden durch Superantigene zur Proliferation sowie zur Freisetzung von Cytokinen und der Cytolyse benachbarter Zellen angeregt. Ausgelöst wird diese unkontrollierte, systemische T-Zellaktivierung durch Bindung der Superantigene an konservierte Bereiche von MHCII-Molekülen bzw. die Vß-Kette des T-Zellrezeptors. Dohlsten et al. (Proc Natl. Acad. Sci. USA 91 (1994), 8945) beschreibt, daß die unspezifische Bindung an MHCII durch eine spezifischere Antigenbindungsstelle eines Antikörpers ersetzt werden kann. Dadurch gelingt es, die T-Zellaktivierung wesentlich kontrollierter ablaufen zu lassen. Durch geeignete Kombination der Antigenspezifitäten eines Antikörpers kann auch hier die Cytolyse-Wirkung der aktivierten T-Zellen auf eine bestimmte Zielzelle begrenzt werden.

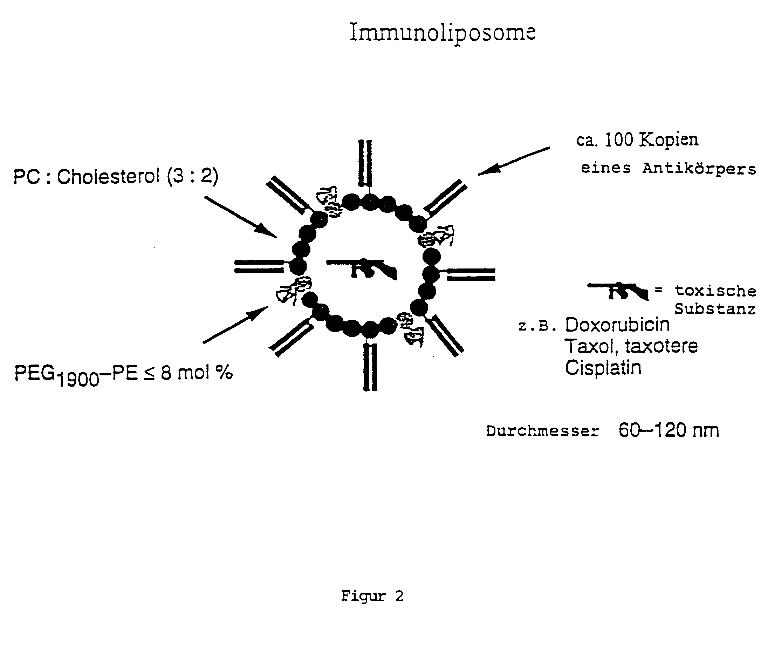

Die Antikörper können auch als membranständige Antikörper in sogenannten Immunoliposomen vorliegen.
Unter Immunoliposomen versteht man Liposomen, die auf ihrer Oberfläche gebunden Antikörper aufweisen (siehe z.B. Phillips et al., J. Immunol. 152 (1994), 3168. Der Aufbau eines sterisch stabilisierten Immunoliposoms ist schematisch in Figur 2 gezeigt. In derartigen Liposomen können cytotoxische Agentien eingeschlossen werden. Durch membranständige Antikörper, die spezifisch eine Zielzelle erkennen, wie oben beschrieben, können diese Liposomen gezielt zu den Zielzellen dirigiert werden. Die durch den membranständigen Antikörper vermittelte hohe Spezifität ist aufgrund der hohen Toxizität der in den Liposomen eingeschlossenen Agentien sehr wichtig.

Bei den oben beschriebenen Antikörpern handelt es sich vorzugsweise um monoclonale, polyclonale, chimäre, rekombinante, synthetische, halbsynthetische oder chemisch modifizierte Antikörper, sowie um Fragmente derartiger Antikörper, insbesondere Fv, Fab, scFv oder F(ab)₂-Fragmente.

Sind die Antikörper für eine in-vivo-Therapie vorgesehen, werden bevorzugt Antikörper oder Derivate oder Fragmente vom Menschen verwendet, oder solche, die derart verändert sind, daß sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig.

Die Herstellung monoclonaler Antikörper, die ihren Ursprung vorzugsweise in Säugetieren, z.B. Mensch, Ratte, Maus, Kaninchen oder Ziege haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfre (Meth. Enzymol. 73 (1981), 3) beschrieben sind.
Die Herstellung polyclonaler Antikörper ist ebenfalls beschrieben, beispielsweise in Harlow und Lane (s.o.).
Ferner ist es möglich, die beschriebenen Antikörper mittels rekombinanter DNA-Technologie nach dem Fachmann geläufigen Techniken herzustellen (siehe z.B. Kurucz et al., J. Immunol. 154 (1995), 4576; Holliger et al., Proc. Natl. Acad. Sci. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und Rekombinante identifiziert und isoliert, die Antikörper mit beiden Spezifitäten produzieren.

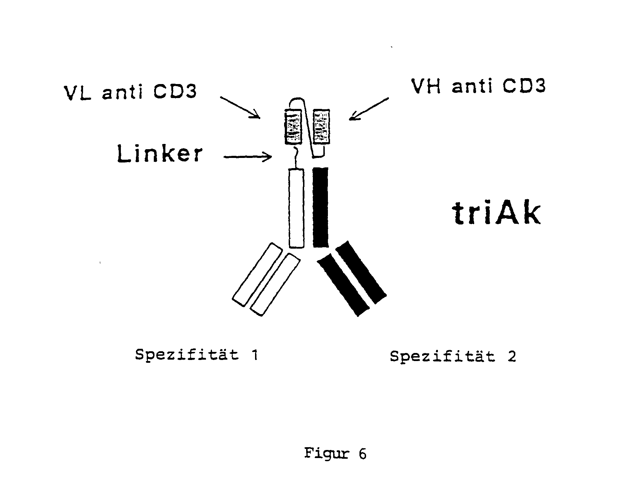

Die Herstellung von Antikörpern mit drei Spezifitäten, sogenannten trispezifischen Antikörpern, kann beispielsweise derart erfolgen, daß an eine der schweren Ig-Ketten eines bispezifischen Antikörpers eine dritte Antigenbindungsstelle mit einer weiteren Spezifität, z.B. in Form eines "single chain variable fragments" (scFv) angekoppelt wird. Das scFv kann beispielsweise über einen

-S-S(G₄S)ₙD-I-Linker

an eine der schweren Ketten gebunden sein (S = Serin, G = Glycin, D = Aspartat, I = Isoleucin). Figur 6 zeigt schematisch den Aufbau eines derartigen trispezifischen Antikörpers, der das Antigen CD3 mittels des angekoppelten scFv-Fragmentes erkennt.

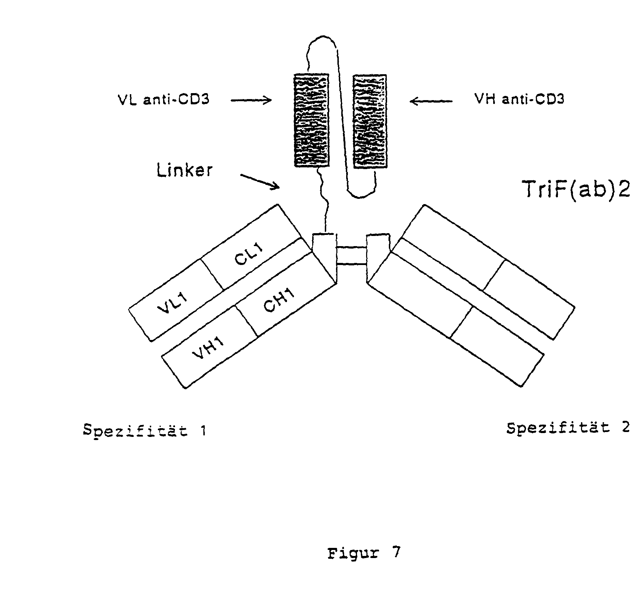

Analog dazu können trispezifische F(ab)₂-Konstrukte hergestellt werden, indem die CH2-CH3-Regionen der schweren Kette einer Spezifität eines bispezifischen Antikörpers ersetzt werden durch ein scFv mit einer dritten Spezifität, während die CH2-CH3-Regionen der schweren Kette der anderen Spezifität beispielsweise durch Einbau eines Stopcodons (am Ende der "Hinge"-Region) in das codierende Gen, z.B. mittels homologer Rekombination entfernt werden (siehe Figur 7).

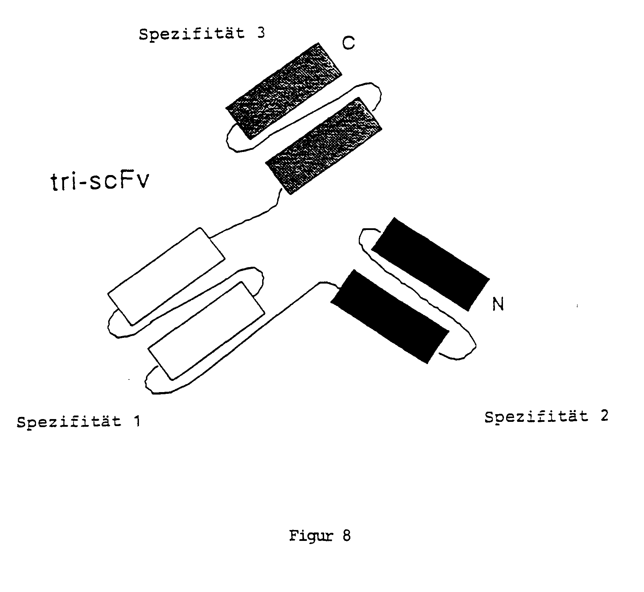

Möglich ist auch die Herstellung trispezifischer scFv-Konstrukte. Hierbei werden drei VH-VL-Regionen, die drei verschiedene Spezifitäten repräsentieren, hintereinander in Reihe angeordnet (siehe Figur 8).

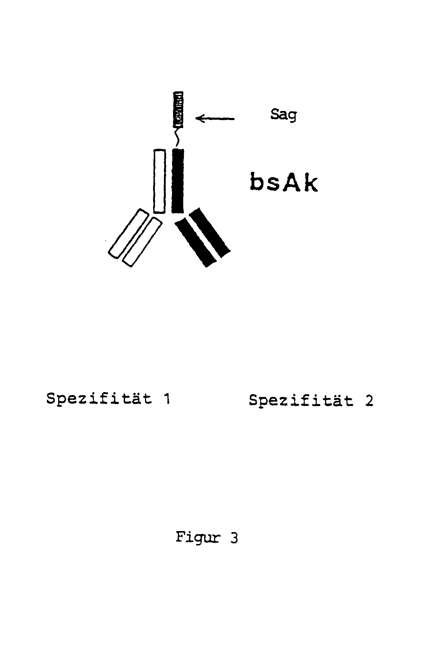

Bei der Herstellung von Antikörpern, die mit einem Superantigen gekoppelt sind, wird beispielsweise eine der schweren Immunglobulinketten eines Antikörpers oder Antikörperfragmentes um eine T-Zell-aktivierende Superantigensequenz verlängert. Dabei kann diese Sequenz z.B. über einen

-S-S(G₄S)ₙD-I-Linker

mit einer der schweren Ig-Ketten verbunden sein. Das Anfügen der Linker- und Superantigensequenzen an die schwere Ig-Kette kann beispielsweise mittels homologer Rekombination auf DNA-Ebene in einer Hybridomazellinie stattfinden (Lang und Mocikat, Mol. Gen. Genet. 242 (1994), 528). Eine derartige Konstruktion eines bispezifischen Antikörpers mit einer Superantigensequenz ist beispielhaft in Figur 3 gezeigt.

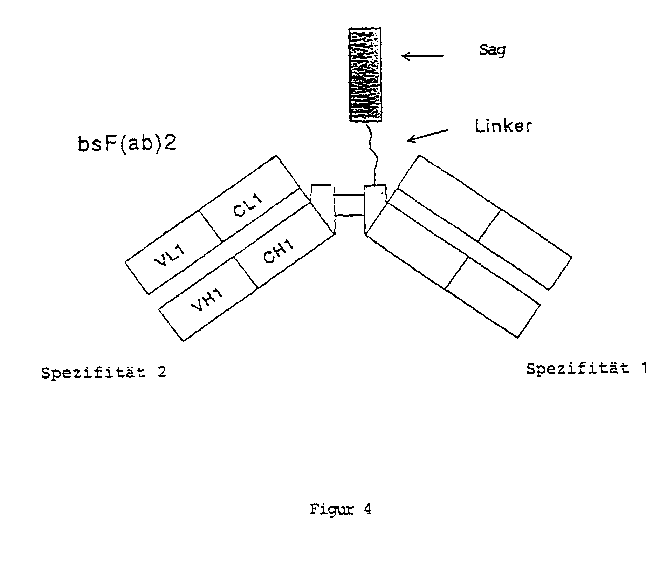

Ferner ist es möglich, F(ab)₂-Fragmente mit einem Superantigen zu koppeln. Dies ist in Figur 4 beispielhaft für ein bispezifisches F(ab)₂-Fragment gezeigt. In diesem Fall werden die CH2, CH3-Regionen einer schweren Immunglobulinkette durch eine Superantigensequenz ersetzt, während die CH2- und CH3-Regionen der anderen schweren Immunglobulinkette durch den Einbau eines Stopcodons mittels homologer Rekombination entfernt werden.

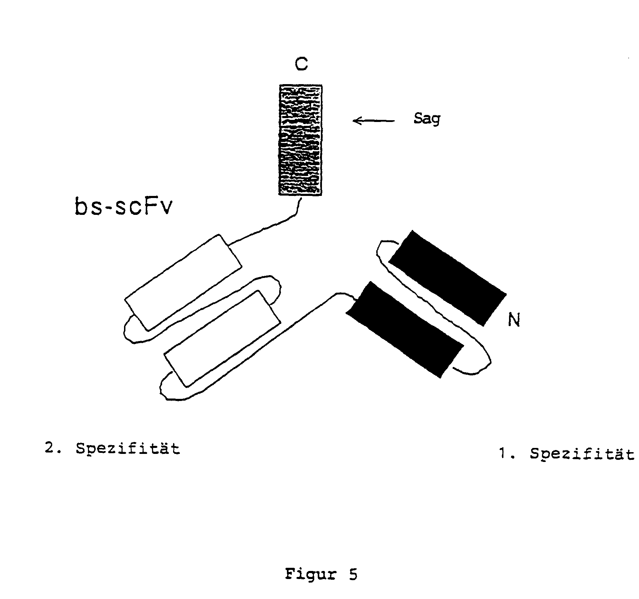

Eine weitere Möglichkeit besteht darin, sogenannte "single chain variable fragments" (scFv) mit einem Superantigen zu koppeln. Dies ist beispielhaft für ein derartiges bispezifisches Fragment in Figur 5 gezeigt. Hierbei werden beispielsweise lediglich die V-Regionen mit verschiedener Spezifität aneinandergekoppelt bei vollständiger Entfernung der konstanten Antikörperregionen und anschließend wird an eine der V-Regionen wie oben beschrieben eine Superantigensequenz gekoppelt.

Neben den beschriebenen Antikörpern kann bei der Herstellung der Arzneimittel gängige, dem Fachmann geläufige pharmazeutisch verträgliche Trägerstoffe verwendet werden.

Ein Therapieschema zur Eliminierung residueller Tumorzellen nach einer MHCII-inkompatiblen Knochenmarkstransplantation mit Hilfe der hergestellten Arzneimittel kann beispiels-weise darin bestehen, daß bereits frühzeitig nach der Knochenmarktransplantation Antikörper verabreicht werden, die spezifisch das MHCII-Antigen des Empfängers erkennen und die eine Eliminierung der Tumorzellen durch ADCC oder Komplement-Mechanismen erlauben, oder Antikörper, die neben dem Empfänger-MHCII-Antigen ein Antigen auf einer Fc-Rezeptor-positiven Effektorzelle (z.B. NK-Zellen, Granulozyten oder Makrophagen) erkennen. Da nach einer Knochenmarktransplantation Fc-Rezeptor-positive Zellen schneller aus dem Spenderknochenmark heranwachsen als T-Zellen, kann mit einer derartigen Behandlung bereits wenige Tage nach der Transplantation begonnen werden. Wenn genügend T-Zellen vom Spendertyp nachgebildet wurden (dies ist der Fall, wenn im peripheren Blut der T-Zellanteil auf über 5 % steigt) können anschließend Antikörper verabreicht werden, die spezifisch das Empfänger MHCII-Antigen und daneben das Antigen CD3 erkennen. Hierdurch werden T-Zellen aktiviert und an die MHCII-exprimierenden Tumorzellen herangeführt, was zu deren Eliminierung führt.
Durch eine derartige Kombination von unterschiedlichen Therapieformen können bereits in einem früheren Stadium, wenn erst wenige überlebende Tumorzellen in dem Patienten existieren, diese effektiv und sehr selektiv mit relativ geringen Mengen an Antikörpern und ohne wesentliche Nebenwirkungen angegriffen werden. Da nach Knochenmarktransplantationen immer noch ein sehr hoher Prozentsatz der Patienten (ca. 40 %) am Leukämierezidiv verstirbt, könnte daher eine derartig milde, nebenwirkungsarme Therapieform bei allen Patienten vorbeugend vorgenommen werden, ohne ein Rezidiv abzuwarten und dadurch die Behandlungsaussichten wesentlich zu verschlechtern.

Die Arzneimittel eignen sich neben der beschriebenen in vivo-Immuntherapie nach MHCII-inkompatibler Knochenmarktransplantation auch zur Immuntherapie nach auto-loger Knochenmarktransplantation und auch generell zur in vivo- und in vitro-Tumordepletion. Durch Kombination einer anti-MHCII- mit beispielsweise einer anti-CD10-Spezifität kann die Selektivität der in den Arzneimitteln enthaltenen Antikörper für Tumorzellen derart gesteigert werden, daß eine Schädigung anderer MHCII-tragender Zellen oder Gewebe fast vollkommen ausgeschlossen ist.

Wie erwähnt, betrifft die Erfindung die Verwendung von Antikörper, die spezifisch das MHCII-Antigen eines zu behandelnden Patienten erkennen, und ihrer bevorzugten Ausführungsformen, sowie die Verwendung von monospezifischen MHCII-erkennenden Antikörpern, zur Herstellung von Arzneimitteln zur in vivo- oder in vitro-Immuntherapie, insbesondere von Tumoren, vorzugsweise von Leukämien und von Tumoren aus entarteten Zellen des Epithels oder Endothels. Von Interesse ist insbesondere die Verwendung zur Herstellung von Arzneimitteln zur Behandlung von B-Zell-Lymphomen, Hodgkin-Lymphomen, anaplastischen "large cell" Lymphomen, adulten T-Zell-Leukämien oder Myelomen.

Die ober beschriebenen Antikörper, sowie monospezifische Antikörper, die das MHCII-Antigen eines Patienten erkennen, können auch zur Unterdrückung einer Host-versus-Graft-Reaktion, zur Unterdrückung einer Autoimmunreaktion oder zur Auslösung einer Immunsuppression verwendet werden.
- Figur 1: zeigt die prinzipielle Wirkungsweise bispezifischer Antikörper
- Figur 2: zeigt schematisch den Aufbau von sogenannten Immunoliposomen
PC = Phosphatidylcholin
PEG = Polyethylenglykol
PE = Phosphatidylethanolamin
- Figur 3: zeigt schematisch einen mit einer Superantigensequenz gekoppelten bispezifischen Antikörper
Sag = Superantigensequenz
bsAK = bispezifischer Antikörper
- Figur 4: zeigt schematisch ein bispezifisches F(ab)₂-Su-perantigenkonstrukt
Sag = Superantigensequenz
bsF(ab)₂ = bispezifisches F(ab)₂-Fragment
- Figur 5: zeigt schematisch ein bispezifisches "single chain variable fragment"-Superantigen-Konstrukt.
Sag = Superantigensequenz
bs-scFv = bispezifisches "single chain variable fraqment"
- Figur 6: zeigt schematisch einen trispezifischen Antikörper, bei dem ein "single chain variable fragment", das CD3 erkennt, über einen Linker an eine der schweren Ketten eines bispezifischen Antikörpers gekoppelt wurde. triAK = trispezifischer Antikörper
- Figur 7: zeigt schematisch ein trispezifisches F(ab)₂-Konstrukt, bei dem eine schwere Kette eines bispezifischen Antikörpers durch ein scFv-Fragment ersetzt wurde, das CD3 erkennt, und bei dem die andere schwere Kette entfernt wurde.
- Figur 8: zeigt schematisch ein trispezifisches scFv-Konstrukt
- Figur 9: zeigt die Ergebnisse einer Untersuchung, in der den in Beispiel 1 beschriebenen Balb/c-Mäusen BCL1-Zellen injiziert werden.
HB3 = Antikörper gegen den MHCII-Haplotyp I-A^{d}
BiC = bispezifischer Antikörper mit den Spezifitäten anti-I-A^{d} und anti-CD3
Wie aus dieser Figur ersichtlich ist, konnte eine signifikante Steigerung der Überlebensrate bei Mäusen beobachtet werden, denen MHCII-spezifische Antikörper injiziert wurden.

Das Beispiel erläutert die Erfindung.

### Beispiel 1

Um die Nutzbarkeit des MHCII-Antigens als operationelles, tumorspezifisches Antigen zu prüfen, das die spezifische Eliminierung von MHCII-exprimierenden Tumorzellen erlaubt, wurden Balb/c (I-A^{d}+)/C57BL/6 (I-A^{b}+) chimären Mäusen (MHCI-und MHCII-inkompatibel) 2 x 10⁴ BCL1-Zellen (B-Zell-Lymphom, I-A^{d}+) injiziert.

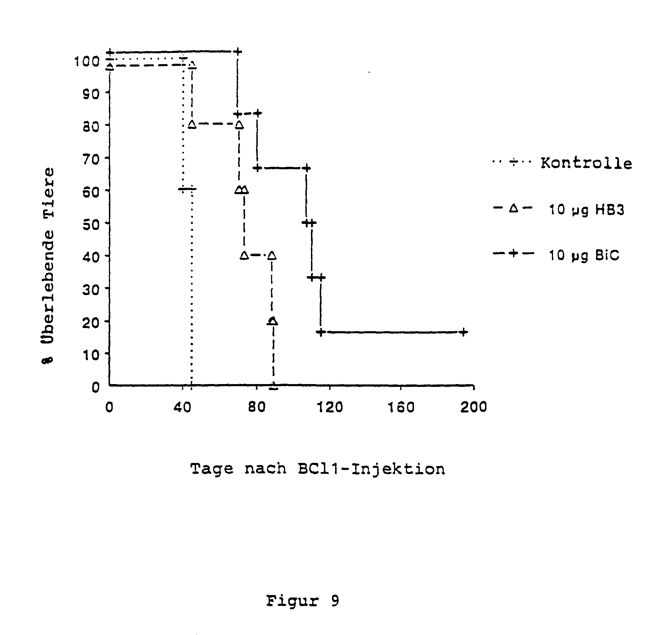

Vier Tage später wurde den behandelten Mäusen 10 *µ*g anti-I-A^{d}(MHCII)-Antikörper injiziert. Bei derart behandelten Mäusen konnte eine signifikante Verlängerung (log rank, p = 0,04) der Überlebenszeit beobachtet werden, im Vergleich zu Kontrollmäusen, denen 2 x 10⁴ BCL1-Tumorzellen, jedoch kein Antikörper injiziert wurde (siehe Fig. 9).

## Patentansprüche

1. Verwendung eines monospezifischen Antiköpers der spezifisch das MHCII-Antigen eines zu behandelnden Patienten erkennt, oder eines Antikörpers mit zwei oder mehr Spezifitäten, wobei mindestens eine Spezifität das MHCII-Antigen eines zu behandelnden Patienten erkennt, zur Herstellung eines Arzneimittels zur Eliminierung residueller Tumorzellen nach einer MHCII-inkompatiblen Knochenmarktransplantation.

2. Verwendung nach Anspruch 1, wobei der Antikörper mindestens eine weitere Spezifität aufweist, die ein Antigen auf einer Effektorzelle erkennt.

3. Verwendung nach Anspruch 2, wobei es sich bei dem Antigen der Effektorzelle um CD1, CD2, CD3, CD4, CD5, CD6, CD7, CD8, CD11b, CD13, CD16, CD28, CD32, CD33, CD40L, CD45R, CD56, CD64 oder einen IL-2-Rezeptor handelt.

4. Verwendung nach Anspruch 2 oder 3, wobei es sich bei den Effektorzellen um T-Zellen, Granulocyten, Monocyten, Makrophagen, NK (natural killer)-Zellen, Mast-Zellen oder Langerhans-Zellen handelt.

5. Verwendung nach einem der Ansprüche 1 bis 4, wobei der Antikörper mindestens eine weitere Spezifität aufweist, die ein Antigen erkennt, das auf Leukämiezellen, Zellen der Hämotopoese oder entarteten Zellen des Epithels oder Endothels exprimiert wird.

6. Verwendung nach Anspruch 5, wobei die weitere Spezifität eines der folgenden Antigene erkennt:
CD1, CD2, CD3, CD4, CD5, CD6, CD7, CD8, CD10, CD11, CD11b, CD13, CD14, CD19, CD20, CD21, CD22, CD23, CD24, CD30, C333, CD37, CD40, CD41, CD44v3, CD44v6, CD45R, CD56, CD71, B220, Ig-Idiotyp, einen IL-2-Rezeptor, einen IL-6-Rezeptor oder ein tumorassoziiertes Antigen.

7. Verwendung nach einem der Ansprüche 1 bis 6, wobei es sich bei dem Antikörper um einen monoclonalen, rekombinanten, (halb) -synthetischen oder chemisch modifizierten Antiköroer oder um ein Fragment eines solchen Antikörpers handelt.

8. Verwendung nach einem der Ansprüche 1 bis 7, wobei der Antikörper gekoppelt ist mit einem Enzym, einer toxischen Substanz, einem Radionuclid, Biotin oder einem Superantigen.

9. Verwendung nach einem der Ansprüche 1 bis 8, wobei es sich bei den Tumorzellen um Leukämiezellen handelt.

10. Verwendung nach Anspruch 9, wobei es sich bei der Leukämie um ein B-Zell-Lymphom, ein Hodgkin-Lymphom, ein anaplastisches "large cell" Lymphom, eine adulte T-Zell-Leukämie oder ein Myelom handelt.

11. Verwendung nach einem der Ansprüche 1 bis 8, wobei es sich bei den Tumorzellen um entartete Zellen des Epithels oder Endothels handelt.

## Claims

1. Use of a monospecific antibody which specifically recognises the MHCII-antigen of a patient to be treated or of an antibody having two or more specificities, wherein at least one specificity recognises the MHCII-antigen of a patient to be treated for the production of a pharmaceutical composition for eliminating residual tumour cells after a MHCII-incompatible bone marrow cell transplantation.

2. The use of claim 1, wherein the antibody exhibits at least one additional specificity which recognises an antigen on an effector cell.

3. The use of claim 2, wherein the antigen of the effector cell is CD1, CD2, CD3, CD4, CD5, CD6, CD7, CD8, CD11b, CD13, CD16, CD28, CD32, CD33, CD40L, CD45R, CD56, CD64 or an IL-2-receptor.

4. The use of claim 2 or 3, wherein the effector cells are T-cells, granulocytes, monocytes, macrophages, NK (natural killer) cells, mast cells or Langerhans cells.

5. The use of any one of claims 1 to 4, wherein the antibody exhibits at least one additional specificity which recognises an antigen which is expressed on leukaemia cells, cells of the haematopoiesis or degenerate cells of the epithelium or endothelium.

6. The use of claim 5, wherein the further specificity recognises one of the following antigens:
CD1, CD2, CD3, CD4, CD5, CD6, CD7, CD8, CD10, CD11, CD11b, CD13, CD14, CD19, CD20, CD21, CD22, CD23, CD24, CD30, CD33, CD37, CD40, CD41, CD44v3, CD44v6, CD45R, CD56, CD71, B220, Ig-idiotype, an IL-2-receptor, an IL-6-receptor or a tumour associated antigen.

7. The use of any one of claims 1 to 6, wherein the antibody is a monoclonal, recombinant, (semi)-synthetic or chemically modified antibody or a fragment thereof.

8. The use of any one of claims 1 to 7, wherein the antibody is coupled with an enzyme, a toxic substance, a radionuclide, biotin or a super-antigen.

9. The use of any one of claims 1 to 8, wherein the tumour cells are leukaemia cells.

10. The use of claim 9, wherein the leukaemia is a B-cell lymphoma, a Hodgkinlymphoma, an anaplastic large cell lymphoma, an adult T-cell leukaemia or a myeloma.

11. The use of any one of claims 1 to 8, wherein the tumour cells are degenerate cells of the epithelium or endothelium.

## Revendications

1. Utilisation d'un anticorps monospécifique reconnaissant spécifiquement l'antigène MHCII d'un patient à traiter, ou d'un anticorps à deux spécificités ou plus, au moins une spécificité reconnaissant l'antigène MHCII d'un patient à traiter, pour la fabrication d'un médicament destiné à l'élimination de cellules tumorales résiduelles après une transplantation de moelle osseuse incompatible avec MHCII.

2. Utilisation selon la revendication 1, dans laquelle l'anticorps présente au moins une autre spécificité qui reconnaît un antigène sur une cellule effectrice.

3. Utilisation selon la revendication 2, dans laquelle, en ce qui concerne l'antigène de la cellule effectrice, il s'agit de CD1, CD2, CD3, CD4, CD5, CD6, CD7, CD8, CD11b, CD13, CD16, CD28, CD32, CD33, CD40L, CD45R, CD56, CD64 ou d'un récepteur d'lL-2.

4. Utilisation selon la revendication 2 ou 3, dans laquelle, en ce qui concerne les cellules effectrices, il s'agit de lymphocytes T, granulocytes, monocytes, macrophages, cellules NK (tueuses naturelles), mastocytes ou de cellules de Langerhans.

5. Utilisation selon l'une des revendications 1 à 4, dans laquelle l'anticorps présente au moins une autre spécificité qui reconnaît un antigène qui est exprimé sur des cellules leucémiques, des cellules de l'hématopoïèse ou des cellules dégénérées de l'épithélium ou de l'endothélium.

6. Utilisation selon la revendication 5, dans laquelle l'autre spécificité reconnaît l'un des antigènes suivants :
CD1, CD2, CD3, CD4, CD5, CD6, CD7, CD8, CD10, CD11, CD11b, CD13, CD14, CD19, CD20, CD21, CD22, CD23, CD24, CD30, CD33, CD37, CD40, CD41, CD44v3, CD44v6, CD45R, CD56, CD71, B220, un idiotype d'lg, un récepteur d'IL-2, un récepteur d'IL-6 ou un antigène associé à une tumeur.

7. Utilisation selon l'une des revendications 1 à 6, dans laquelle, en ce qui concerne l'anticorps, il s'agit d'un anticorps monoclonal, recombinant, (semi)-synthétique ou modifié chimiquement, ou d'un fragment d'un tel anticorps.

8. Utilisation selon l'une des revendications 1 à 7, dans laquelle l'anticorps est couplé à une enzyme, une substance toxique, un radionuclide, la biotine ou un superantigène.

9. Utilisation selon l'une des revendications 1 à 8, dans laquelle, en ce qui concerne les cellules tumorales, il s'agit de cellules leucémiques.

10. Utilisation selon la revendication 9, dans laquelle, en ce qui concerne la leucémie, il s'agit d'un lymphome à lymphocytes B, d'un lymphome d'Hodgkin, d'un lymphome anaplasique à "grandes cellules", d'une leucémie à lymphocytes T de l'adulte ou d'un myélome.

11. Utilisation selon l'une des revendications 1 à 8, dans laquelle, en ce qui concerne les cellules tumorales, il s'agit de cellules dégénérées de l'épithélium ou de l'endothélium.
